# EUROPEAN PATENT APPLICATION

(11) **EP 1 396 545 A1**
(43) Date of publication of application: **10.03.2004**
(21) Application number: 02733272.5
(22) Date of filing: 03.06.2002
(51) Int. Cl.: C12N 15/54, C12N 5/10, C12P 21/02, C12Q 1/48, C07K 14/47, C07K 16/40, G01N 33/68, A61K 45/00, A61K 38/00, A61P 35/00, A61P 17/02, A61P 43/00

(54) **CELL CYCLE REGULATORY FACTOR**

(30) Priority: 04.06.2001 JP 2001168792
(71) Applicant: TAIHO PHARMACEUTICAL CO., LTD., Tokyo 101-0054 (JP); Nakanishi, Makoto, Nagoya-shi, Aichi 468-0023 (JP)
(72) Inventor: NAKANISHI, Makoto, Nagoya-shi, Aichi 468-0023 (JP)
(74) Representative: Jones, Helen Marjorie Meredith
(86) International application number: PCT/JP2002/005411
(87) International publication number: WO 2002/099110

(57) **Abstract**

The present invention provides:
a protein according to (i) or (ii) below:
   (i) a protein comprising the amino acid sequence of SEQ ID NO: 1; or
   (ii) a protein comprising the amino acid sequence of SEQ ID NO: 1, in which one or more amino acids are replaced, deleted, inserted, and/or added, and which has kinase activity; and
a gene comprising the DNA of (i) or (ii) below:
   (i) a DNA comprising the base sequence of SEQ ID NO: 2; or
   (ii) a mammal-derived DNA encoding a protein that hybridizes to a DNA comprising the base sequence of SEQ ID NO: 2 and has kinase activity.

## Description

### TECHNICAL FIELD

The present invention relates to a mammal-derived protein involved in the regulation of the cell cycle, and the gene thereof. In particular, the invention is directed to a gene that codes for a novel human protein Cdr2 (Changed division response 2) involved in cell cycle regulation and that has homology to Cdr1/Nim1 and Cdr2, both of which are yeast cells proliferation regulating factors; a recombinant vector containing such a gene; a transformant obtained from the vector; a process for producing human Cdr2 (hCdr2) using the gene; and recombinant hCdr2 obtained according to the process.

### BACKGROUND OF THE INVENTION

Cell division and proliferation include processes such as gene replication and mitosis, thereby forming the cell cycle. The cell cycle of a normal eukaryotic cell is divided into four stages (G1, S, G2, M, in turn) according to the cell form and biochemical activity. A cell that has deviated from the cell cycle is referred to as being in the G0 phase or an aphasic state. When cells in the normal cell cycle actively replicate, DNA replication occurs in the S phase and cell division occurs in the M phase. The progression of the cell cycle is strictly controlled by cell cycle checkpoints. For example, when DNA is damaged, mitosis halts until the damaged DNA is repaired (*Science* 246 (1989): 629-634; *Science* 274 (1996): 1664-1672). Usually, a DNA damage reaction route halts the cell cycle by inhibiting the cyclin-dependent kinase activity. In human cells, the delay of the G2 phase due to DNA damage is greatly dependent on the inhibitory phosphorylation of Cdc2 (*Mol. Cell Biol.* (1997), 8:1-11; *J. Cell Biol.* 134 (1996): 963-970). Therefore, this delay is likely to be caused by the activity change in the inhibitory kinase and phosphatase that act on Cdc2. However, it has not been evidenced that activities of these enzymes significantly change in response to DNA damage (*Cancer Res.* (1997) 57: 5168-5178).

In human cells, three types of Cdc25 proteins are expressed. Cdc25A is especially necessary in transition from the G1 phase to the S phase (*EMBO. J.,* 13 (1994): 4302-4310; *EMBO*. *J.,* 13 (1994): 1549-1556). Cdc25B and Cdc25C are needed in transition from the G2 phase to the M phase (*J. Cell Sci.,* 7 (1996): 1081-1093; *Cell,* 67 (1991): 1181-1194; *Proc. Natl. Acad. Sci. Acd. USA,* 88 (1991): 10500-10504; *J*. *Cell Biol*., 138 (1997): 1105-1116). The exact involvement of Cdc25B and Cdc25C in the progression of the M phase is not known.

Recent years have seen the discoveries of cell cycle regulating factors thought to play an important role in cell cycle checkpoints (*Science* 277 (1997): 1450; *Cell* 91 (1997): 865). For example, through a study using a bakers' yeast (*Saccharomyces cerevisiae*), scCds1 (also called Rad53 or Chk2) having a kinase activity was identified as a cell cycle regulating factor (*Genes & Development* 8 (1994): 2401; *Genes & Development* 10 (1996): 395). Furthermore, spCds1, a homologue of scCds1, was identified in a fission yeast (*Schizosaccharomyces pombe*) (*Nature* 374 (1995): 817; *Genes* & *Development* 12 (1998): 382). Accordingly, functions of cell cycle regulating factors are being gradually revealed.

This mechanism is thought to be accomplished by transmitting a deactivation signal caused by DNA damage to a factor involved in cell cycle progress. The mechanism of signal transmission elucidated so far is that an ATM (ataxia telangiectasia) kinase or the like is activated by DNA damage; molecules such as Chk1 and Chk2 are phosphorylated and then activated; in G2 arrest, Chk1 and Chk2 promote the phosphorylation of Serine 216 of the dephosphorylation enzyme, Cdc25C; 14-3-3 proteins bind; and migration of Cdc25C to the nucleus is inhibited. G2 arrest is thought to occur because Cdc25C is an enzyme activating an intra-nuclear enzyme Cdc2 kinase that controls the transition to the M phase and, as a result, the activation of Cdc2 is inhibited. Recently, it has been reported that Chk2 enhances the activation of the p53 cancer suppressor gene protein, and the transcription factor p53 induces the expression of the cyclin-dependent kinase inhibitor p21waf-1, thereby promoting G1 arrest.

It is suggested that p53 controls the expression of factors such as GADD45 and the like involved in G2 arrest. Based on, these facts, kinases such as Chk1, Chk2, etc., are presumed to control the activity of various factors and be involved in the cell cycle arrest when DNA is damaged.

The mechanism of cell cycle arrest is important in providing time to repair DNA damage. Due to such a repair mechanism, cancer cells can escape DNA damage caused by irradiation or anti-cancer agents used especially in cancer treatment. Therefore, when this checkpoint mechanism is destroyed, the effectiveness of irradiation and anti-cancer agents used against cancer cells can be increased.

### DISCLOSURE OF THE INVENTION

A primary object of the present invention is to provide a mammal-derived gene that has homology to spCdr1/Nim1 and spCdr2, and a protein encoded by such a gene.

The inventors found that a novel enzyme discovered in the process of analyzing the functions of Chk2 is involved in cell proliferation and can be used as an important means for developing pharmaceuticals used in diagnosing and treating proliferative disorders such as cancer, rheumatism, etc. The present invention was accomplished based on the above findings.

Specifically, the inventors produced a rabbit polyclonal antibody against human cell cycle regulatory factor Chk2, in order to analyze the functions of Chk2. In the process of analyzing the phosphorylation of Chk2 caused by a variety of DNA damage by Western blotting using the polyclonal antibody, they found that one of the proteins that react with the polyclonal antibody is phosphorylated by DNA damage.

The inventors presumed that those proteins that record changes in the state of phosphorylation caused by DNA damage are involved in signal transmission of intracellular reactions to respond to DNA damage. Therefore, they cloned the protein gene that responds to the antibody and analyzed its functions. In particular, they separated the gene clone detected by the antibody using the lambda phage human cDNA expression library and determined its gene sequence, and as a result, succeeded in isolating the full-length gene of human Cdr2 that is homologous to yeast Cdr2. Yeast Cdr2 inhibits the deactivation of Cdc2 by phosphorylating the Cdc2 inactive enzyme, Wee1 kinase. Therefore, when Cdr2 is deactivated, Cdc2 is deactivated by active Wee1 as a result, inducing G2 arrest. In this connection, the inventors discovered that the protein hCdr2 of the present invention is mainly expressed in the S phase and is, as with Chk1 and Chk2, a protein kinase that can phosphorylate Cdc25.

Therefore, the instant protein is considered to regulate the cell cycle, and can be used as an important means for developing novel pharmaceuticals for diagnosing and treating proliferative disorders, such as cancer and the like. In particular, compounds that inhibit the activity and expression of the instant protein are expected to be used as anti-cancer agents.

The invention relates to a novel kinase protein of mammalian origin involved in cell cycle regulation; the gene thereof; molecules used in the detection, isolation, and production of the protein and the gene; and a method for screening a compound that regulates the activity of the protein. The invention provides a mammal-derived gene that has homology to spCdr1/Nim1 and spCdr2, and the protein thereof. Moreover, the invention provides a method for preparing a vector that can be used in producing such a protein; a transformant; and a recombinant protein. Furthermore, the invention provides an oligonucleotide used in detecting and isolating the gene, and an antibody used in detecting and purifying the protein.

Additionally, the present invention provides a method for screening a compound that binds to the protein and a compound that enhances or inhibits the activity of the protein. In particular, the present invention provides the following:
Item 1. A protein according to (i) or (ii) below:
   (i) a protein comprising the amino acid sequence of SEQ ID NO: 1; or
   (ii) a protein comprising the amino acid sequence of SEQ ID NO: 1, in which one or more amino acids are replaced, deleted, inserted, and/or added, and which has kinase activity.
Item 2. A protein that is encoded by a mammal-derived DNA that hybridizes to a DNA comprising the base sequence of SEQ ID NO: 2, and has a kinase activity.
Item 3. A protein defined in Item 1 or 2 exhibiting a phosphorylating activity to Cdc25.
Item 4. A protein defined in Item 1 or 2 exhibiting a phosphorylating activity to serine 216 of Cdc25c.
Item 5. A protein defined in Item 1 or 2 exhibiting a phosphorylating activity to serine 309 of Cdc25B.
Item 6. A gene encoding the protein of (i) or (ii) below:
   (i) a protein comprising the amino acid sequence of SEQ ID NO: 1; or
   (ii) a protein comprising the amino acid sequence of SEQ ID NO: 1, in which one or more amino acids are replaced, deleted, inserted, and/or added, and which has kinase activity.
Item 7. A gene comprising the DNA of (i) or (ii) below:
   (i) a DNA comprising the base sequence of SEQ ID NO: 2; or
   (ii) a mammal-derived DNA encoding a protein that hybridizes to a DNA comprising the base sequence of SEQ ID NO: 2 and has kinase activity.
Item 8. An expression product of the gene defined in Item 6 or 7.
Item 9. An expression vector for a recombinant comprising the gene defined in Item 6 or 7.
Item 10. A transformant transformed by the expression vector for the recombinant defined in Item 9.
Item 11. A method for producing a recombinant protein, the method comprising culturing the transformant of Item 10 and purifying the resulting protein.
Item 12. An oligonucleotide specifically hybridizing to the gene of Item 6 or 7 and comprising at least 15 nucleotides.
Item 13. An oligonucleotide comprising at least 15 nucleotides that specifically hybridizes to the gene of Item 6 or 7 and can inhibit the expression of the gene.
Item 14. An antibody capable of binding to the protein according to any of Items 1 to 5 or to the expression product according to Item 8.
Item 15. A method for screening a compound that binds to the protein defined in any of Items 1 to 5 or to the expression product defined in Item 8, the method comprising the steps of:
   (a) contacting a test sample with the protein defined in any of Items 1 to 5 or the expression product defined in Item 8; and
   (b) selecting a compound that binds to the protein defined in any of Items 1 to 5 or to the expression product defined in Item 8.
Item 16. A method for screening a compound that can promote or inhibit kinase activity of the protein defined in any of Items 1 to 5 or the expression product defined in Item 8, the method comprising the steps of:
   (a) contacting the protein defined in any of Items 1 to 5 or the expression product defined in Item 8 with a corresponding substrate in the presence of test compounds;
   (b) assaying the substrate-targeted kinase activity of the protein defined in any of Items 1 to 5 or the expression product defined in Item 8; and
   (c) selecting a compound that can promote or inhibit the substrate-targeted kinase activity of the protein defined in any of Items 1 to 5 or the expression product defined in Item 8, by comparing the activity with that obtained in an assay in the absence of the test compound (control).
Item 17. A compound that can be isolated by the method of Item 15 or 16.
Item 18. A compound defined in Item 17, wherein the compound is a protein.
Item 19. A compound defined in Item 18, wherein the protein is an antibody.
Item 20. A pharmaceutical composition comprising a compound that inhibits kinase activity of the protein defined in any of Items 1 to 5 or the expression product defined in Item 8.
Item 21. A pharmaceutical composition comprising a compound that inhibits in a cell the expression of the gene defined in Item 6 or 7.
Item 22. A pharmaceutical composition according to Item 20 or 21, wherein the pharmaceutical composition is an anti-cancer agent.
Item 23. A use of the pharmaceutical composition defined in any of Items 20 to 22 to obtain a pharmaceutical for cancer treatment.
Item 24. A method for treating a cancer comprising administering to a cancer patient a therapeutically effective amount of the pharmaceutical composition defined in any of Items 20 to 22.
Item 25. A pharmaceutical composition comprising a compound that promotes kinase activity of the protein defined in any of Items 1 to 5 or the expression product defined in Item 8.
Item 26. A pharmaceutical composition comprising a compound that promotes in a cell the expression of the gene defined in Item 6 or 7.
Item 27. A pharmaceutical composition defined in Item 25 or 26, wherein the pharmaceutical composition is a vulnerary.
Item 28. A use of the pharmaceutical composition defined in any of Items 25 to 27 to obtain a pharmaceutical for healing a wound.
Item 29. A method for healing a wound comprising administering to a patient having a wound a therapeutically effective amount of the pharmaceutical composition defined in any of Items 25 to 27.

The present invention primarily relates to a mammal-derived novel protein involved in cell cycle regulation.

The amino acid sequence of a human-origin protein, termed "hCdr2", that is included in the protein of the invention is shown in SEQ ID NO: 1, and the base sequence of the cDNA coding for the protein is shown in SEQ ID NO: 2. The hCdr2 protein isolated by the present inventors has a significant homology to cell cycle regulating factors spCdr2 (*Cell* 49 (1987): 569) and spCdr1 (*Molecular Biology of the Cell* 9 (1998): 3399 and 3321) known to be present in yeasts.

This fact suggests that hCdr2 functions in cell cycle regulation in the same manner as these yeast-derived proteins do, and that the kinase activity of the protein plays a vital role with respect to this function. Moreover, such a relationship between the hCdr2 protein and cell cycle regulation suggests that the protein, its gene and, in addition, compounds that regulate the functions of the hCdr2 protein can be applied to diagnosing and treating cell cycle disorders such as cancer and the like.

According to methods known to those skilled in the art, the protein of the invention can be produced using genetic engineering techniques. A recombinant protein can be produced, for example, by incorporating a DNA encoding the instant protein (for example, a DNA comprising the base sequence of SEQ ID NO: 2) into a suitable expression vector, introducing this vector into host cells, and purifying the protein from the transformant thus obtained. A natural protein may be obtained, for example, by preparing a column in which an antibody obtained by immunizing a small animal with the recombinant protein is immobilized, and conducting affinity chromatography using the column on a tissue or cell extract expressing the protein of the present invention.

Moreover, the present invention provides proteins functionally equivalent to the hCdr2 protein. In order to isolate such proteins, methods including introducing a mutation into the amino acids of the protein are well known to those skilled in the art. As methods known in the art for modifying amino acids, examples include those described in *Shin Saibo-Kogaku Jikken Protocol* (New Cell Engineering Protocols), pp 241-248, edited by the Carcinostatic Research Division, Institute of Medical Science, Tokyo University, 1993.

Through the use of the commercially available "QuickChange Site-Directed Mutagenesis Kit" (produced by Stratagene), isolating a protein that is functionally equivalent to the hCdr2 protein of SEQ ID NO: 1 is generally conducted by suitably replacing an amino acid that does not impair the function of the hCdr2 protein. Amino acid mutations sometimes occur spontaneously. Therefore, the protein of the present invention includes proteins that are functionally equivalent to the hCdr2 protein, in which one or more amino acids of the amino acid sequence (SEQ ID NO: 1) of the hCdr2 protein are substituted, deleted, inserted, and/or added.

The term "insert(ed)" herein refers to that an amino acid is added to a site other than both terminals of the aforementioned amino acid sequence. The term "add(ed)" means that an amino acid is added to both terminals of the aforementioned amino acid sequence.

The term "functionally equivalent" protein herein refers to a protein that has a kinase activity equivalent to that of the natural hCdr2 protein. The "kinase activity" in the present invention refers to an activity that produces a phosphorylated protein by transferring the phosphate group (-PO₃H₂) to a serine, threonine, or tyrosine residue of the substrate protein (e.g., Wee1 and the like). Protein kinase activities can be detected according to the method described hereunder in the examples. The number of amino acids mutated in a protein that is functionally equivalent to the hCdr2 protein is not limited insofar as the kinase activity equivalent to that of the hCdr2 protein is not impaired. Normally, it is no more than 50 amino acids, preferably no more than 20 amino acids, more preferably no more than 10 amino acids, and still more preferably no more than a few amino acids; specifically it is no more than 5 amino acids. Mutation sites are not limited insofar as the kinase activity equivalent to that of the hCdr2 protein is not impaired.

Furthermore, examples of the protein of the invention include those that are polypeptides having a specific homology to the amino acid sequence of SEQ ID NO: 1 and that are functionally equivalent to the hCdr2 protein. The specific homology refers to not less than 70%, preferably not less than 80%, more preferably not less than 90%, and most preferably not less than 95%. The scope of the invention includes those having such a homology.

Another method known to one skilled in the art for isolating a functionally equivalent protein is a hybridization technique (for example, Sambrook, J et al., *Molecular Cloning 2*^{*nd*} *ed.* (1989), Cold Spring Harbor Laboratory Press, 9.47-98, 58). Specifically, one skilled in the art can usually isolate DNA having a high homology to part or the entire DNA (SEQ ID NO: 2) coding for the hCdr2 protein and produce a protein that is functionally equivalent to the hCdr2 protein using such DNA. As described, the examples of the instant protein include those that are encoded by DNA hybridizing to DNA that encodes the hCdr2 protein and are functionally equivalent to the hCdr2 protein.

The term "functionally equivalent" proteins as used herein refers, as described above, to those proteins that exhibit a kinase activity equivalent to that of the hCdr2 protein.

Examples of organisms other than humans for isolating functionally equivalent proteins include mice, rats, dogs, rabbits, and monkeys. Proteins derived from mammals other than humans are useful for developing animal model systems for pharmaceutical development.

"Stringent conditions" for hybridization to isolate DNA that codes for functionally equivalent proteins consist of 10% formamide, 5x SSPE, 1x Denhardt's solution, and 1x salmon sperm DNA. More preferable (more stringent) conditions are 25% formamide, 5x SSPE, 1x Denhardt's solution, and 1x salmon sperm DNA. However, there are several other factors to be considered besides the formamide concentration described above that influence the stringent conditions for hybridization. One skilled in the art can suitably select these factors to achieve an identical stringency. Moreover, instead of hybridization, it is possible to isolate DNA that encodes functionally equivalent proteins by gene amplification methods, e.g., polymerase chain reaction (PCR), using a portion of the DNA (SEQ ID NO: 2) coding for the hCdr2 protein as a primer. Advantageously, such proteins can be produced according to a recombination method or a synthesis method, such as PCR cloning mechanisms, which generally include therein steps of preparing a pair of primers consisting of about 15 to 50 nucleotides with respect to the gene site to be cloned; contacting these primers with human cell-derived mRNA, cDNA, or genomic DNA; conducting the PCR method under conditions in which amplification of the desired site occurs (reverse transcription should be conducted at the beginning as necessary); isolating the amplified domain or fragment; and recovering the amplified DNA. The techniques described herein are known to those skilled in the art as they are described, for example, in Sambrook et al., "Molecular Cloning" *a Laboratory Manual,* Cold Spring Harbor Laboratory Press, 1989; and *Current* *Protocols in Molecular Biology,* Supplement 46, John Wiley & Sons, Inc., April 1999.

The mammal-derived DNAs coding for proteins that are functionally equivalent to the "hCdr2" protein and that are isolated by these hybridization or gene amplification techniques usually exhibit a significant homology to the DNA coding for the human-derived hCdr2 protein of SEQ ID NO: 2. The term "significant homology" refers to a sequence identity of no less than 70%, preferably no less than 80%, more preferably no less than 90%, and most preferably no less than 95%. To calculate a homology, methods known in the art can be used, such as the BLAST method, FASTA method, and the like. For example, the method described in the publication (*Proc. Natl. Acad. Sci. USA 80* (1983): 726) can be used.

The invention further relates to a gene that codes for the protein described above. The gene of the invention is not limited insofar as it codes for the protein of the invention, and examples thereof include, in addition to double-stranded DNA, single-stranded DNA such as sense strands and antisense strands consisting of such double-stranded DNA. They also include cDNA, genomic DNA, and chemically synthetic DNA, etc.

In particular, such genes include:
a gene that encodes a protein comprising the amino acid sequence of SEQ ID NO: 1;
a gene that encodes a protein comprising the amino acid sequence of SEQ ID NO: 1, in which one or more amino acids are replaced, deleted, inserted, and/or added, and which has kinase activity;
a gene that comprises DNA comprising the base sequence of SEQ ID NO: 2;
a gene that comprises mammal-derived DNA coding for a protein that hybridizes to DNA comprising the base sequence of SEQ ID NO: 2 and has kinase activity; and
a gene comprising a polynucleotide having a specific homology with respect to the polynucleotide coding for a protein comprising the amino acid sequence of SEQ ID NO: 1 or the polynucleotide comprising the base sequence of SEQ ID NO: 2. The phrase "polynucleotide having a specific homology" refers to a homology of at least 50%, preferably not less than 70%, and more preferably not less than 90%. The scope of the present invention includes those having such a homology.

The gene of the invention can be isolated by using methods usually employed by those skilled in the art, for example, by screening cDNA libraries or genomic libraries using a portion or all of the base sequence disclosed in SEQ ID NO: 2 as a probe, or by the PCR method using a portion or all of the base sequence as a template.

The gene (DNA) of the invention is of use for producing as a recombinant protein the protein of the invention in large amounts. Such a recombinant protein is produced by inserting a gene (for example, the DNA of SEQ ID NO: 2) that encodes the protein of the invention into a suitable expression vector, introducing the vector into a suitable host cell, culturing the resulting transformant, and purifying the protein thus expressed.

As a host-vector system for producing a recombinant protein, numerous systems known to those skilled in the art may be used. Host cells are not limited and include prokaryotic cells of *E*. *coli* and the like, and eukaryotic cells of yeast, animal cells, insect cells, etc. Vectors for expressing a recombinant protein within cells preferably include, for example, although varying according to the host cell, pGEX (manufactured by Pharmacia) and pET (manufactured by Novagen) when *E. coli* is used; pcDNA3.1 (manufactured by Invitrogen) when animal cells are used; pVL1392 (manufactured by Invitrogen) and the Bac-to-Bac baculovirus expression system (manufactured by GIBCO-BRL) when insect cells are used. Expression vectors may include replication-origin, selection markers, promoters, RNA splice sites, polyadenylation signals, etc.

Vectors can be introduced into cells according to methods known in the art, for example, electroporation, the calcium phosphate method, lipofection, the DEAE dextran method, and the like.

Culturing of the transformant and separation and purification of the recombinant protein expressed in the transformant can be conducted according to standard methods. When pGEX (produced by Pharmacia) is used as a vector, the expressed recombinant protein (fusion protein) can be readily purified by glutathione sepharose affinity chromatography. When pET (produced by Novagen) is used, it is readily purified by nickel agarose affinity chromatography.

The scope of the present invention includes the expression products of the aforementioned gene. Particularly, the scope of the invention includes, in addition to the protein described above, those specifically modified by a host cell, for example, sugar chain binding, phosphorylated materials, etc.

The gene of the invention may be applied to gene therapy. Since the gene of the present invention is involved in cell cycle regulation, the target diseases of such gene therapy are proliferative disorders, such as cancer and the like. When the gene of the present invention is used for gene therapy, it is implanted into a vector for expressing the gene in the human body and introduced into the body by injecting it *in vivo* or *ex vivo* according to retroviral, liposomal, adenoviral, and like methods.

Furthermore, the present invention is directed to an antibody that binds to the protein of the invention. The form of the antibody of the invention is not limited, and examples include polyclonal and monoclonal antibodies. Chimeric antibodies, humanized antibodies, and human antibodies are also included. Furthermore, examples include not only the complete form of antibodies, but also Fab fragments, F(ab')2 fragments, single-chain scFv, and the like. Antibodies of the invention can be prepared according to methods known to those skilled in the art. Polyclonal antibodies can be prepared according to known methods, for example, in which the protein of the invention is injected into a rabbit, and the immunoglobulin fraction is purified using ammonium sulfate precipitation. Monoclonal antibodies can be produced, for example, by preparing a hybridoma between myeloma cells and spleen cells of a mouse immunized with the protein of the invention and collecting the monoclonal antibody secreted in the culture medium, as well as by injecting the hybridoma into the abdominal cavity to obtain the monoclonal antibody in large quantities. The antibody thus prepared can be used for the detection and affinity purification of the protein of the invention, and can also be applied to the diagnosis and antibody treatment of cell proliferative disorders, such as cancer and the like, caused by expression abnormalities of the instant protein. When these antibodies are to be used for antibody treatment, humanized antibodies or human antibodies are preferable in view of antigenicity.

Moreover, the present invention features an oligonucleotide that comprises at least 15 nucleotides and specifically hybridizes to the gene that codes for the instant protein. The term "oligonucleotide" refers to an oligonucleotide or an analogue thereof that is produced from a naturally existing base and a sugar portion bonded by original phosphodiester bonding. Therefore, the first group of compounds that fall within the definition of the term includes naturally occurring species, or synthetic species prepared from naturally occurring subunits or homologues of these subunits. The subunits refer to the base-sugar combination bound by phosphodiester bonding or other bonding with respect to adjacent subunits. The second group of the oligonucleotide includes the homologues thereof. They function identically to the oligonucleotide, but contain a residue having a portion not occuring naturally. The second group includes oligonucleotides chemically modified at the phosphoric acid group, sugar portion, or 3',5'-terminal to increase stability. Examples include oligophosphorothioate in which one of the oxygen atoms of the phosphodiester group between the nucleotides is substituted with sulfur, oligomethylphosphonate in which -CH₃ is substituted, and the like. The phosphodiester bonding can be substituted by other non-ionic and non-chiral structures. Furthermore, as oligonucleotide homologues, modified base forms, i.e., species containing purine and pyrimidine that are not found naturally, can be used. The "oligonucleotides" herein include peptide nucleic acid (PNA, see, for example, *Bioconjugate Chem.,* Vol. 5, No. 1, 1994) in addition to DNA and RNA.

The phrase "to specifically hybridize" used herein indicates that significant cross-hybridization does not occur with genes that code for other proteins under normal hybridizing conditions, preferably under stringent conditions. The "stringent conditions" are as described above. Such oligonucleotides can be used as a probe to detect and isolate the gene encoding the instant protein and also as a primer for amplification.

The present invention, moreover, relates to an oligonucleotide having at least 15 nucleotides that specifically hybridizes to the gene of the present invention and can inhibit the expression of the gene. In particular, examples of the oligonucleotide include oligonucleotides that specifically bind to the DNA of the instant gene and inhibits transcription; and oligonucleotides that bind to a specific site of the mRNA of the instant gene and inhibits translation. Such "oligonucleotides" include DNA, RNA, PNA, etc. These oligonucleotides may be of a natural type or of a modified type such that they can be efficiently incorporated into cells. For example, the oxygen atom of a phosphate bond found in the natural type can be substituted with a sulfur atom. The antisense and triplet methods using such oligonucleotides can be conducted according to Murray, J.A.H., *Antisense RNA and DNA*, WileyLiss, Inc., New York (1992): 1-49.

In the present invention, while transcription initiation sites, translation initiation sites, exon-intron junction sites, and 5'cap sites are preferable as the target sites for the mRNA to which an oligonucleotide hybridizes, sites that do not cause steric hindrance can also be selected in view of the secondary structure of the mRNA.

With respect to the target sites of DNA to which the oligonucleotide of the present invention hybridizes, transcription regulatory factors may be excluded by binding to the regulatory region of the instant gene.

The especially preferable embodiment of the invention includes the use of an oligonucleotide that hybridizes to the base sequence encoding the amino acid sequence of SEQ ID NO: 1, and inhibits the expression of the hCdr2 protein.

The oligonucleotide of the invention can be prepared according to synthesis methods known to those skilled in the art, for example, solid-phase synthesis using a synthesizing device produced by AppliedBiosystems. Using this method, oligonucleotide homologues, e.g., phosphorothioate and alkylation derivatives can be produced (Akira Murakami et al., "Chemical Synthesis of Functional Antisense DNA", *Organic synthetic chemistry* 48(3), 1990: 180-193).

Furthermore, the present invention relates to a method for screening a compound that binds to the protein and expression product of the invention. The instant screening method includes the steps of (a) contacting a test sample with the protein and expression product of the invention and (b) selecting a compound having binding activity to the protein and expression product of the invention.

Examples of test samples used in screening include, although are not limited to, purified proteins (including antibodies), expression products of gene libraries, synthetic peptide libraries, cell extracts, cell culture supernatants, synthetic low-molecular-weight compound libraries, and the like. Methods for selecting a compound that has binding activity to the protein and expression product of the present invention include those known in the art, for example, the use of a BIAcore, affinity column wherein the protein and expression product of the invention are solid-phased, and the like.

As a method for isolating a compound that binds to the protein (including the expression product) of the invention, the following method is known to those skilled in the art. A protein that binds to the instant protein can be prepared, for example, by creating a cDNA library from cells presumed to express a protein that binds to the instant protein using a phage vector (λ gt11, ZAP, etc.); expressing this cDNA library on LB-agarose; fixing the expressed proteins on a filter; biotin-labeling the instant protein or purifying it as a fusion protein with a GST protein; reacting the protein with the above-described filter; and detecting plaques expressing the binding proteins using streptavidin or an anti-GST antibody (West-Western Blotting method) (Skolnik EY, Margolis B, Mohammadi M, Lowenstein E, Fischer R, Drepps A, Ullrich A, and Schlessinger J, "Cloning of PI3 Kinase-Associated p85 Utilizing a Novel Method for Expression/Cloning of Target Proteins for Receptor Tyrosine Kinases.", *Cell* 65 (1991): 83-90). A protein that binds to the protein of the invention can also be prepared using two-hybrid systems ("MATCHMAKER Two-Hybrid System", "Mammalian MATCHMAKER Two-Hybrid Assay Kit" and "MATCHMAKER One-Hybrid System" (all of which produced by Clontech) and "HybriZAP Two-Hybrid Vector System" (produced by Stratagene). See Dalton S, and Treisman R, "Characterization of SAP-1, a Protein Recruited by Serum Response Factor to the c-fos Serum Response Element." *Cell* 68 (1992): 597-612.

In the two-hybrid systems, first, the protein of the invention is fused to the SRF or GAL4 binding region and expressed in yeast cells; a cDNA library, expressed in a form such that this library fuses with the VP16 or GAL4 transcriptional activation region, is prepared from cells presumed to express a protein that binds to the protein of the invention; and this cDNA library is introduced into the yeast cells described above. Then, cDNA derived from such a library is isolated from the positive clones detected (when a protein that binds to the instant protein is expressed in yeast cells, the binding of the respective proteins activates a reporter gene, thereby enabling positive clones to be detected). Thereafter, the isolated cDNA is introduced into *E. coli,* and the protein expressed by the cDNA is purified to obtain a protein encoded by the cDNA.

A protein that binds to the instant protein can also be prepared by introducing the culture supernatants or cell extracts of cells that are considered to express a protein that binds to the instant protein onto an affinity column in which the instant protein is immobilized; and purifying the protein that binds specifically to the column. By analyzing the amino acid sequence of the protein thus obtained, synthesizing an oligo DNA based on the analysis and screening a cDNA library using this DNA as a probe, DNA that codes for a protein that binds to the instant protein is obtainable.

Other methods known to those skilled in the art for isolating low-molecular-weight compounds, proteins (or the genes thereof), and peptides that bind to the instant protein include a method of screening molecules that bind when the immobilized instant protein is exposed to synthetic compounds, natural substance library, or random phage peptide display libraries; and a high-throughput screening method using combinatorial chemistry techniques (*Science* 273 (1996): 458-464; *Nature* 384 (1996): 11-13; and Nature 384 (1996): 17-19).

Compounds that bind to the protein of the invention obtained by such methods serve as candidate compounds for pharmaceutical agents that promote or inhibit the activity of the protein. Intracellular proteins that bind to the instant protein are considered to be closely involved in the cell cycle regulating function, specifically the kinase activity, of the instant protein. Therefore, if an intracellular protein that binds to the protein of the invention is obtained, it enables the development of pharmaceuticals for disorders caused by abnormal cell cycle regulation, such as cancer and the like, by screening for compounds that inhibit the binding of the respective proteins.

Furthermore, the present invention relates to a method for screening a compound that promotes or inhibits the activity of the protein of the invention. The screening method of the invention comprises the steps of (a) contacting the protein (including its expression product) of the invention with a corresponding substrate in the presence of a test compound, (b) assaying the substrate-targeted kinase activity of the protein, and (c) selecting a compound that promotes or inhibits the substrate-targeted kinase activity of the protein by comparing its activity with the activity assayed in the absence of the test compound (control). Test compounds used for screening are not limited and examples thereof include synthetic low-molecular-weight compound libraries, purified proteins (including antibodies), expression products of gene libraries, synthetic peptide libraries, cell extracts, cell culture supernatants, etc.

Examples of substrates used in detecting the kinase activity of the instant protein include, although are not limited to, Cdc25 (such as Cdc25A, Cdc25B and Cdc25C; especially Cdc25B and Cdc25C) and fragments thereof. The contact reaction between the instant protein and the substrate can be conducted, for example, as follows:

Prepare a buffer solution containing a test compound, the instant protein, a substrate, and phosphorus-radiolabeled ATP; after reacting the instant protein and the substrate for a suitable amount of time, detect kinase activity. Kinase activity can be assayed by detecting the radioactivity of the phosphorus bound to the substrate. Radioactivity can be detected by separating the solution thus reacted by electrophoresis (SDS-PAGE), drying the gel, and detecting the phosphorylated substrate band by autoradiography. For a control experiment, kinase activity is similarly assayed without adding the test compound. Based on the comparison with the control, compounds that significantly enhance or inhibit kinase activity are then selected. A compound thus obtained that significantly enhances a kinase activity becomes a candidate for a promoter of the instant protein and contributes to the development of pharmaceuticals, such as vulneraries and the like.

A compound that significantly lowers kinase activity becomes a candidate for an inhibitor of the protein of the invention, enabling the development of pharmaceuticals against diseases caused by cell cycle regulation defects such as cancer and the like.

Drugs that specifically inhibit the hCdr2 pathway in cancer cells (those that inhibit the hCdr2 gene expression) can be isolated, for example, by contacting candidate compounds with cancer cell lines, detecting hCdr2 gene expression, comparing the gene expression with that of a control wherein candidate compounds are not contacted, and selecting compounds that comparatively lower the gene expression.

The hCdr2 gene expression can be detected as follows: if it is in the transcriptional product, known methods such as Northern blotting can be employed; and if in the translational product, known methods, such as Western blotting, can be employed. Detection of hCdr2 gene expression can be conducted based on the reporter activity by introducing a vector containing a reporter gene ligated downstream of the promoter of the hCdr2 gene into a cell.

The scope of the present invention encompasses a pharmaceutical composition comprising a compound that inhibits kinase activity of the instant protein and a pharmaceutical composition comprising a compound that inhibits the expression of the instant protein, these pharmaceutical compositions preferably being anti-cancer agents.

Further, the scope of the present invention encompasses a pharmaceutical composition comprising a compound that promotes kinase activity of the instant protein and a pharmaceutical composition comprising a compound that promotes the expression of the instant protein, these pharmaceutical compositions preferably being vulneraries.

When the compound obtained by the screening method of the invention is used as a pharmaceutical for humans and other mammals such as mice, rats, guinea pigs, rabbits, chicken, cats, dogs, sheep, pigs, cattle, monkeys, baboons and chimpanzees, the isolated compound itself can be directly administered to a patient; it can also be administered after formulating it according to known pharmaceutical methods. For example, the compound can be administered orally as tablets that are sugar coated as required, capsules, elixirs, and microcapsules; or parenterally in the form of injections of aseptic solutions or suspensions with water or other pharmaceutically acceptable solutions. The compound may be formulated in suitable combination with, for example, pharmacologically acceptable carriers or media, specifically, sterilized water, physiological saline solution, vegetable oils, emulsifiers, suspending agents, surfactants, stabilizers, flavoring agents, excipients, vehicles, preservatives, binders, etc., in a unit dose form required for generally accepted pharmaceutical practice. The amount of active ingredients in these preparations should be within the range such that a suitable prescribed volume can be obtained.

Examples of additives admixed in tablets and capsules are gelatin, corn starch, tragacanth gum, gum arabic, and like binders; crystalline cellulose and like excipients; corn starch, gelatin, alginic acid, and like swelling agents; magnesium stearate and like lubricants; sucrose, lactose, saccharin, and like sweeteners; and peppermint, *Gaultheria adenothrix* oil, cherry, and like flavorings. When pharmaceuticals are prepared in the form of capsules, a liquid carrier such as oil can be contained in addition to the ingredients described above. Sterile injection compositions can be formulated according to standard pharmaceutical practice using vehicles such as distilled injection solvents.

Examples of aqueous injection solvents include physiological saline and isotonic solutions containing glucose or other adjuvants such as D-sorbitol, D-mannose, D-mannitol, sodium chloride, etc. These can be used in combination with suitable solubilizers, such as alcohols, specifically ethanol, polyalcohols, e.g., propylene glycol and polyethylene glycol; and non-ionic surfactants, such as polysorbate 80 (TM) and HCO-50.

Examples of oleaginous liquid include sesame oil and soy bean oil, which may contain benzyl benzoate or benzyl alcohol as a solubilizer. The oleaginous liquid may further contain buffers such as a phosphate buffer and sodium acetate buffer; pain killers such as procaine hydrochloride; stabilizers such as benzyl alcohol and phenol; and antioxidants. Prepared injection solvents are usually charged into suitable ampules.

Other than intra-arterial, intravenous and subcutaneous injections, administration to patients is conducted intranasally, bronchially, intramuscularly, percutaneously, and orally according to methods known to those skilled in the art. One skilled in the art can suitably select the dosage according to the body weight and age of a patient and the administration method. If the compound can be encoded by DNA, gene therapy can be carried out by implanting the DNA into a gene therapy vector. Although dosage and administration method differ according to the body weight, age, and symptom of a patient, these can be suitably selected by one skilled in the art.

The dosage of the compound described above, although it varies according to the symptoms, is usually about 0.1 mg to about 100 mg per day for an adult (body weight 60 kg) when the compound is administered orally, preferably about 1.0 mg to about 50 mg, and more preferably about 1.0 mg to about 20 mg.

When the compound is administered parenterally, dosage per administration varies according to the patient, targeted organ, symptom, and administration method. When the compound is administered in injection form to an adult (body weight 60 kg), it is effective to intravenously inject in an amount of about 0.01 mg to about 30 mg per day, preferably about 0.1 mg to about 20 mg, and more preferably about 0.1 mg to about 10 mg. Additionally, in the case of administering to other animals, the compound is administered in an amount calculated from the amount described above based on the 60-kg body weight or based on the body surface area typically calculated with such a body weight.

### BRIEF DESCRIPTION OF THE DRAWINGS

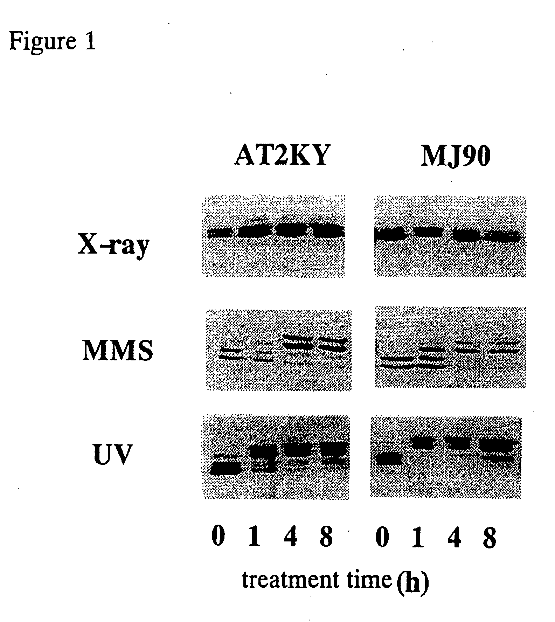

Figure 1 shows the results of analyzing cell proteins, to which various treatments are conducted to damage the DNA, by Western blotting using an anti-hChk2 antibody. Shown are two different types of protein (protein of the present invention), one exhibits a behavior different from that of Chk2 observed using the anti-hChk2 polyclonal antibody. In Chk2, band shifts were observed that are considered to result from the phosphorylation induced by the varied DNA damage. In contrast, in the other protein (protein of the invention) that the anti-hChk2 polyclonal antibody recognizes, band shifts caused by UV and MMS treatments were observed in the AT2KY cells.
Figure 2 (Figures 2-A to 2-C) shows the amino acid sequence and cDNA sequence of hCdr2 of the present invention.
Figure 2-A displays the region in the cDNA sequence corresponding to the 1^{st} to 900^{th} bases.
Figure 2-B continues from Figure 2-A and exhibits the region in the cDNA sequence corresponding to the 901^{st} to 1,800^{th} bases.
Figure 2-C continues from Figure 2-B and shows the region in the cDNA sequence corresponding to the 1,801^{th} to 2,799^{th} bases.
Figure 3 (Figures 3-A to 3-C) shows the amino acid sequence of hCdr2 aligned with the amino acid sequences of spCdr2 and spCdr1 of a fission yeast. The amino acid sequences are depicted in one-letter codes. Hyphens indicate amino acid gaps. Positions in which the amino acids of the three proteins are identical are marked with asterisks. Positions in which the amino acids of two of the proteins are identical are marked with dots.
Figure 3-A displays the region in the amino acid sequence of hCdr2 corresponding to the 1^{st} to 298^{th} amino acids.
Figure 3-B continues from Figure 3-A and shows the region in the amino acid sequence of hCdr2 corresponding to the 299^{th} to 567^{th} amino acids.
Figure 3-C continues from Figure 3-B and shows the region in the amino acid sequence of hCdr2 corresponding to the 568^{th} to 754^{th} amino acids.
Figure 4 (Figures 4-A and 4-B) shows the amino acid sequence of the N terminal (1 to 295) of hCdr2 aligned with the amino acid sequence of spCdr2 of a fission yeast. The amino acid sequences are depicted in one-letter codes. Hyphens indicate amino acid gaps. Positions in which the amino acids are similar are marked with dots.
Figure 4-A exhibits the region in the amino acid sequence of hCdr2 corresponding to the 1^{st} to 179^{th} amino acids.
Figure 4-B continues from Figure 4-A and shows the region in the amino sequence of hCdr2 corresponding to the 180^{th} to 359^{th} amino acids.
Figure 5 shows the expression of hCdr2 mRNA in human tissues analyzed by Northern blotting.
Figure 6 (Figures 6-A to 6-C) provides the analysis of the change in hCdr2 expression in the cell cycle of A172 human glioblastoma cultured cancer cell lines.
Figure 6-A pictures the expression of hCdr2 analyzed by Northern blotting.
Figure 6-B shows the expression of the housekeeping gene GAPDH (glyceraldehyde-3-phosphate dehydrogenase) analyzed by Northern blotting.
Figure 6-C provides an analysis of the cell cycle according to the flow cytometry method using PI (propidium iodate) staining.
Figure 7 shows the result of measuring by Western blotting the amount of hCdr2 recombinant protein expressed in an insect cell using a baculovirus expression vector.
Figure 8 presents an analysis of the phosphorylation of the Cdc25 protein of hCdr2 to demonstrate that hCdr2 is a protein kinase.
Figure 8-A shows the kinase assays when GST-Cdc25C and GST-Cdc25C S216A are used as substrates.
Figure 8-B shows the kinase assays when GST-Cdc25B and GST-Cdc25B S309A are used as substrates.

### BEST MODE FOR CARRYING OUT THE INVENTION

The present invention is described in more detail with reference to the examples below, but the scope of the invention is not limited to these examples.

### Example 1: Production of anti-hChk2 polyclonal antibody

The human (h)Chk2 gene disclosed in WO99/67369 was subcloned to a baculovirus vector pVL1392 (produced by Invitrogen Corporation) to prepare pVL1392hChk2myc/6His. The hChk2 protein was expressed by co-infecting 10⁵ cells of Sf-9 insect cells with 1 µg of pVL1392hChk2myc/6His and 0.5 µg of linearized baculovirus DNA using the vector kit Baculoglod (produced by PharMingen). The hChk2 protein was purified by Probond Resin (trade name of nickel NTA resin, produced by Invitrogen Corporation). Using this protein as an antigen, a rabbit was immunized according to a standard method. An anti-hChk2 polyclonal antibody was purified from the antiserum obtained from the rabbit using CNBr-activated Sepharose 4B (produced by Amersham Pharmacia Biotech) bound to GST-hChk2 (*Journal of Biological Chemistry* Vol. 274, No. 44 (1999): 31463-31467).

### Example 2: Detection of phosphorylated protein recognized by anti-hChk2 polyclonal antibody

Cell proteins that had been subjected to various DNA-damaging treatment were analyzed by Western blotting using the anti-hChk2 antibody obtained in Example 1. Normal human fibroblast cell line MJ90 and fibroblast cell line AT2KY established from an ataxia telangiectasia patient were irradiated with ionizing radiation (X-ray: 10 Gy) and UV radiation (0.07 J/cm²) and treated with 0.03% methyl methane sulfonate (MMS) for 1, 4, and 8 hours. Cell lysate was prepared from the cells thus obtained by cooling them with ice using an IP kinase buffer (50 mM HEPES, pH 8.0, 150 mM NaCl, 2.5 mM EGTA, 1 mM EDTA, 0.1% Tween 20, 10% glycerol) containing a protease inhibitor (20 µg/ml soybean trypsin inhibitor, 2 µg/ml aprotinin, 5 µg/ml leupeptin, 100 µg/ml phenylmethylsulfonyl fluoride (PMSF)) and a phosphatase inhibitor (50 mM NaF, 0.1 mM Na₃VO₄, 5 mg/ml phosphatase substrate). The centrifugally-separated supernatants of the cell lysate (100 µg) were subjected to electrophoresis in 8% SDS-Polyacrylamide gel, transferred to PVDF film, and Western blotted according to a standard method using the anti-hChk2 polyclonal antibody as a primary antibody.

As a result, a novel protein differing from hChk2 in molecular weight was detected. In this protein, band shifts that are considered to be caused by phosphorylation induced by the varied DNA damage were observed (Figure 1).

Band shifts were observed in Chk2 of a normal cell resulting from phosphorylation induced by DNA-damaging treatment. However, band shifts did not occur in the ATM kinase-deficient cells AT2KY. Therefore, Chk2 is supposedly phosphorylated by the ATM kinase and thereby activated (*Journal of Biological Chemistry* Vol. 274, No. 44 (1999): 31463-31467).

In contrast, in the other protein recognized by the anti-hChk2 polyclonal antibody obtained in Example 1, band shifts that result from the UV and MMS treatments were observed in the AT2KY cells. The inventors presumed that this protein is phosphorylated by kinases other than ATM, and they initiated the cloning of this protein.

### Example 3: Identification of hCdr2 cDNA

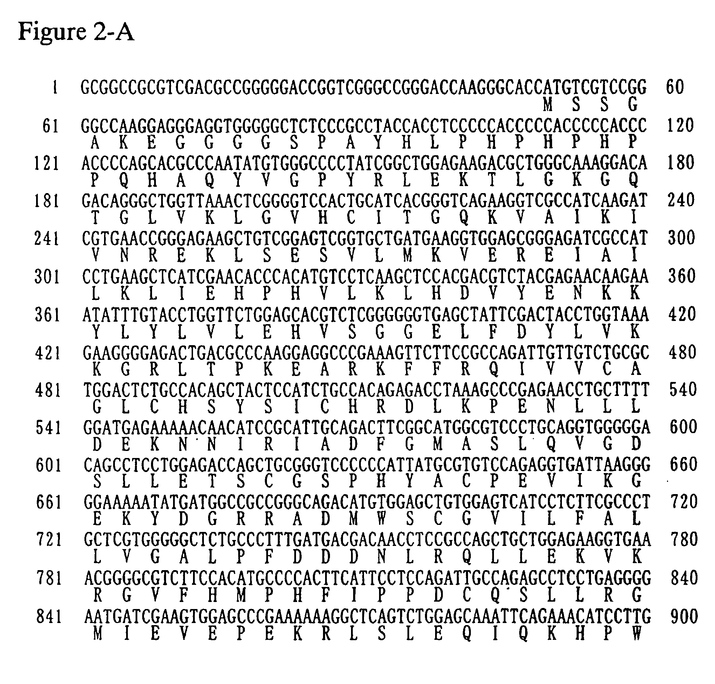

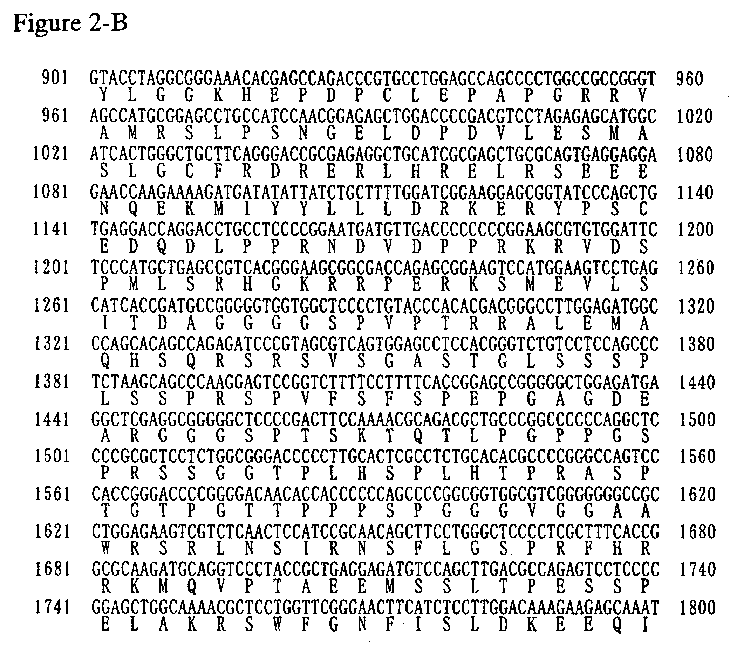

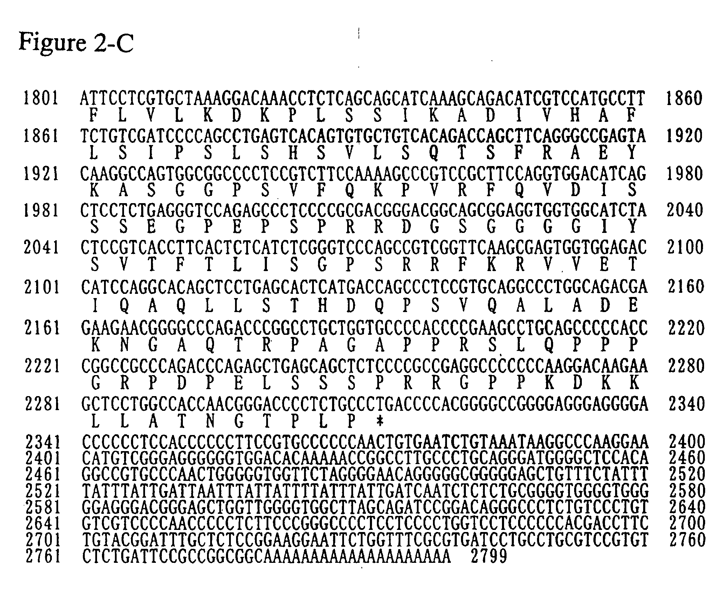

Immunoscreening was conducted using a Human fetal brain cDNA (λ gt11) of Clontech and the anti-hChk2 polyclonal antibody (antibody dilution: 1:2000). Specific details of the screening are described in the instruction manual of the Pico Blue Immunoscreening Kit produced by Stratagene. One of the positive clones thus obtained codes for a gene that has a domain specific to a protein-phosphorylating enzyme. Based on the partial sequence of this gene cDNA, screening was conducted to determine the entire cDNA sequence (SEQ ID NO. 2) according to the hybridization method using the same library as above. The amino acid sequence was estimated based on the cDNA sequence (SEQ ID NO. 1). Figure 2 shows the amino acid sequence and the cDNA sequence.

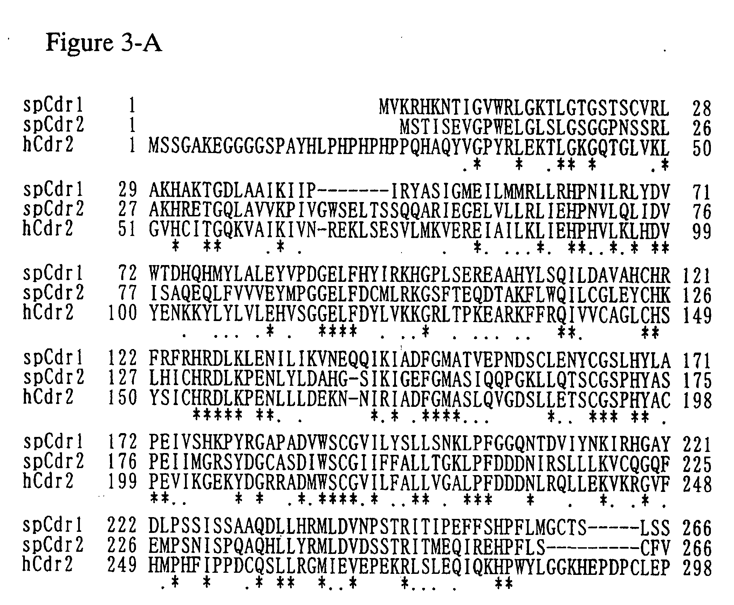

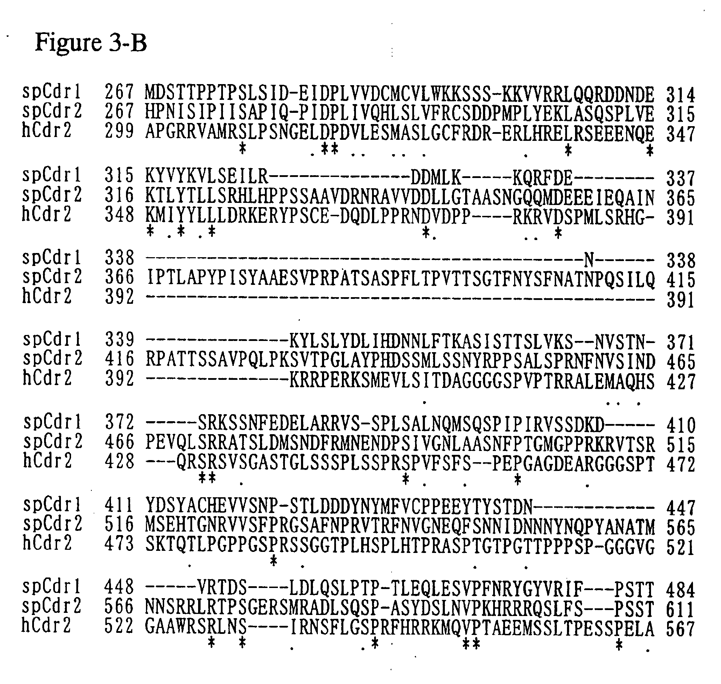

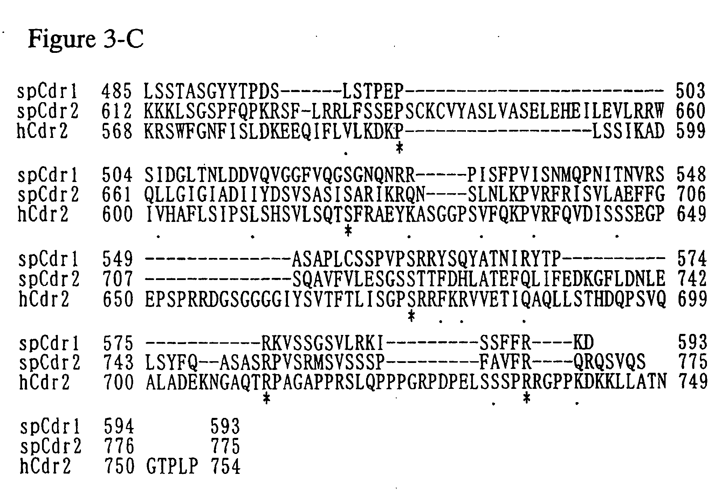

Figure 3 presents the amino acid sequence of human hCdr2 aligned with the amino acid sequences of spCdr2 and spCdr1 of a fission yeast. The amino acid sequences are depicted in one-letter codes. Hyphens indicate amino acid gaps. Positions in which the amino acids of the three proteins are identical are marked with asterisks. Positions in which the amino acids of two of the proteins are identical are marked with dots.

Homologies were discovered among the sequences of hCdr2, spCdr2, and spCdr1. The homology of the amino acid sequence of the entire protein was 28.2% with respect to spCdr2 and 23.9% with respect to spCdr1. In contrast, the homology to hChk2 was 8.4%, thus being low. The inventors presumed that only the epitope region that was recognized by the antibody used in the detection of hCdr2 was analogous.

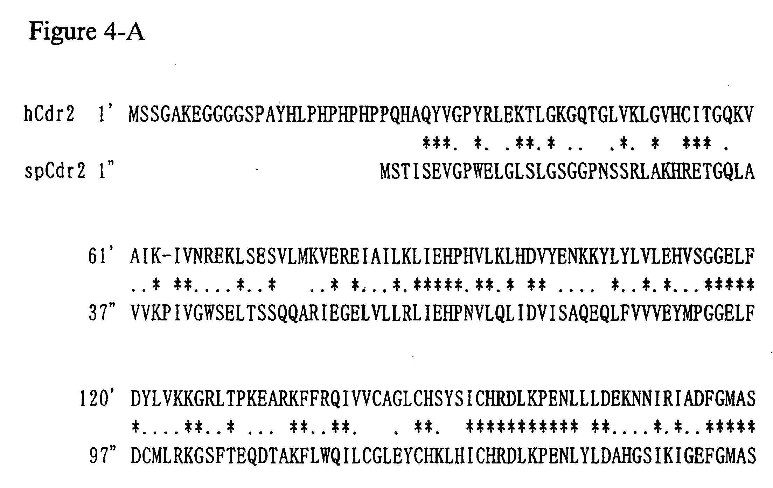

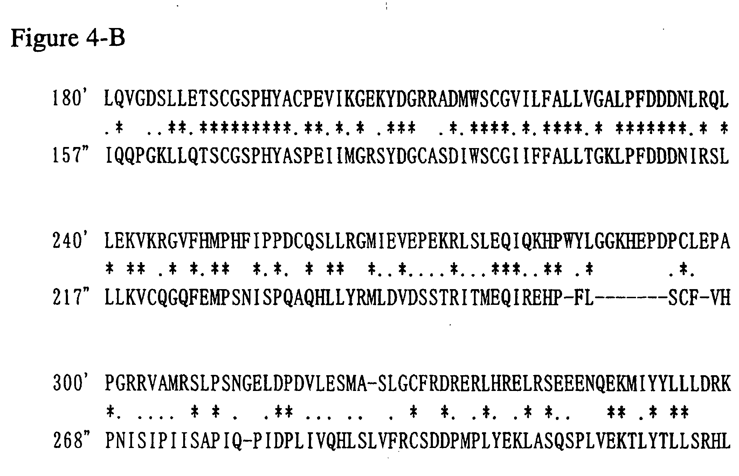

Figure 4 displays the amino acid sequence of the N terminal (1 to 295) of hCdr2 aligned with the amino acid sequence of spCdr2 of a fission yeast. The amino acid sequences are depicted in one-letter codes. Hyphens indicate amino acid gaps. Positions in which the amino acids are similar are marked with dots. A high homology was observed in the N terminal hCdr2 catalytic domain (1-295aa) where the kinase domain is present. Homology was 45.8% to spCdr2 and 35.6% to spCdr1.

### Example 4: Detection by Northern blotting

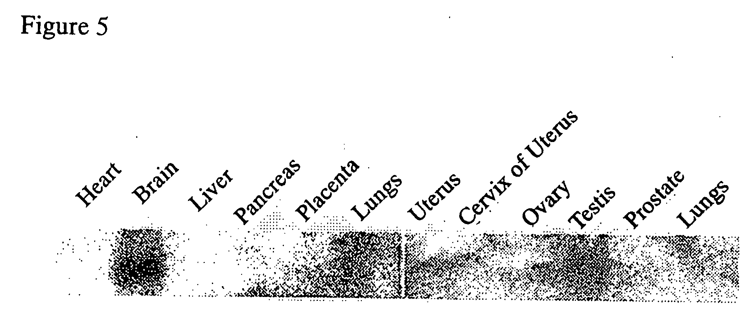

After labeling full-length human cDNA with ³²P, Northern blotting was conducted in a hybridization solution (Ultra Hyb, produced by Ambion Inc.) using a TOYOBO Human normal tissue mRNA blot. Results show strong expression especially in the brain and the testis (Figure 5).

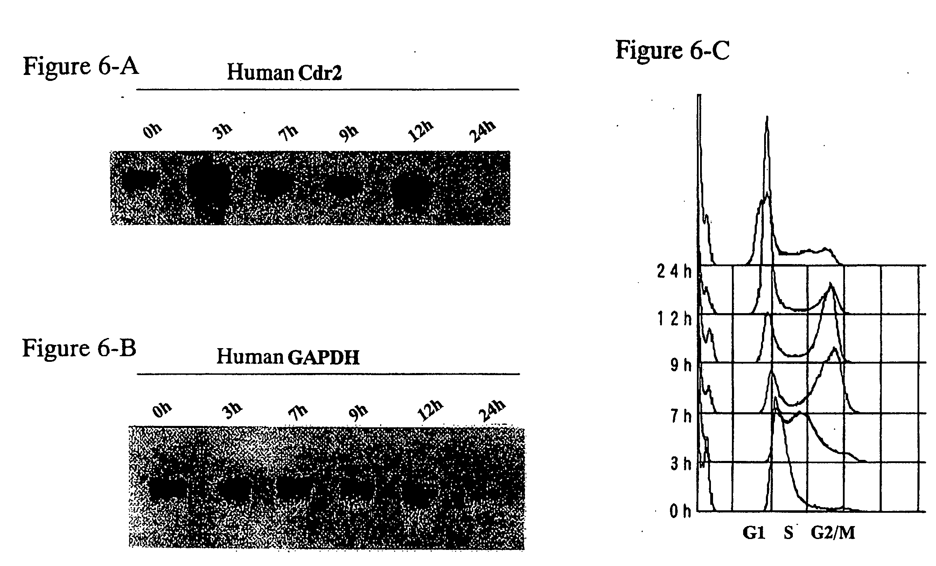

The expression of hCdr2 in HeLa human womb cancer cell line and A172 human glioblastoma cultured cancer cell line was investigated using the RT-PCR method (primer sequence 1: ACTGCATCACGGGTCAGAAGG; primer sequence 2: CGAGACGTGCTCCAGAACCAG). For the RT-PCR method, refer to Sambrook et al., "Molecular Cloning" *A Laboratory Manual*, Cold Spring Harbor Laboratory Press, 1989. Results indicated the detection of transcription products in both cell lines. Subsequently, A172 cells were investigated for cell cycle-dependent expression, in consideration of the function of hCdr2 as a cell cycle regulating factor. The cell cycle of A172 cells was halted at the transition of the G1/S phases by excess thymidine treatment (*Art of Tissue Culture* 5, the Japanese Tissue Culture Association, Asakura Shoten, 1991) and synchronized. A normal culture containing no thymidine was then replaced to continue the cell cycle. Sampling was conducted 0, 3, 7, 9, 12, and 24 hours after release, to prepare mRNA and analyze the cell cycle. Using 10 µg of mRNA obtained at each sampling time, Northern blotting was conducted to analyze the expression of mRNA of hCdr2. To adjust the amount of mRNA assayed among the samples, one of the house keeping genes, GAPDH (glyceraldehyde-3-phosphate dehydrogenase), was used as a marker (Figure 6-B). To analyze the cell cycle at each sampling time, FACS analysis was employed using the Flow cytometry method by PI (propidium iodate) staining. As a result, the greatest expression of hCdr2 was observed in a time period in which most cells were in the S phase (3 hours after release), and it was thus evidenced that the greatest expression is induced in the S phase. Moreover, the amount expressed 24 hours after release was decreased, and therefore expression does not occur much in the G1 phase (Figure 6-A and 6-C).

### Example 5: Detection of protein kinase activity

### (Expression of recombinant protein and kinase assay)

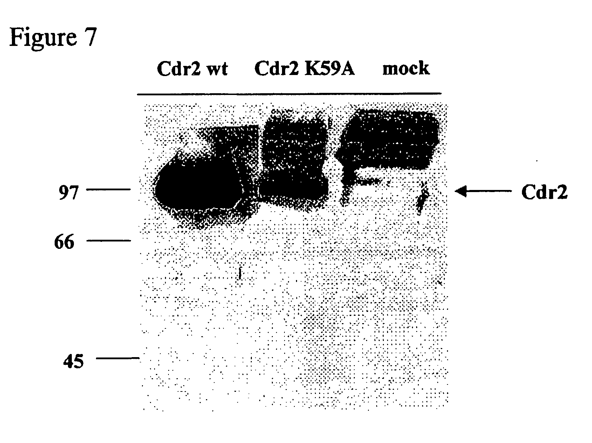

To conduct an *in vitro* kinase assay, an hCdr2 wild-type (wt) and a baculovirus containing cDNA that codes for a kinase-dead mutated protein K59A wherein the 59^{th} amino acid K (lysine) was replaced with A (alanine) were prepared using the Pharmingen Baculovirus Expression Vector System. The recovery of the hCdr2 wt and hCdr2 K59A protein was conducted according to immune precipitation using an anti-c-Myc Tag antibody. Western blotting using an antibody that works against the c-Myc tag present at a terminal of a recombinant protein confirmed that both of the recombinant protein having a size of 90 kD is produced from the baculovirus. In contrast, from a mock infected with a baculovirus containing no hCdr2 gene, no proteins having a molecular weight corresponding to hCdr2 were detected (Figure 7).

GST-fusion recombinant proteins (GST-Cdc25C and GST-Cdc25B) containing human Wee1 (full-length protein), human dephosphorylating enzyme Cdc25C (C terminal peptide: 195-260aa) and human dephosphorylating enzyme Cdc25B (C terminal peptide: 281-381aa) were prepared using *E. coli* for use as assay substrates. When kinase-assaying these recombinant proteins, despite being a human homologue of *S*. *pombe*, the human Wee1 could not be phosphorylated. However, it was revealed that hCdr2 phosphorylates human Cdc25C and Cdc25B, which are cell cycle regulating factors and involved in the M-phase check point. To determine the phosphorylated regions of the Cdc25C and Cdc25B, a peptide fragment containing a 14-3-3 protein binding region was again expressed using *E*. *coli* and kinase-assayed, thereby revealing that the 14-3-3 protein binding region was phosphorylated. The mutant substrate GST-Cdc25C S216A wherein the 216^{th} serine of the Cdc25C amino acid sequence, which is necessary in 14-3-3 protein binding, was replaced with alanine and the mutagenesis substrate GST-Cdc25B S309A wherein the 309^{th} serine of the Cdc25B amino acid sequence was replaced with alanine were prepared according to the PCR method using a primer obtained by mutating the desired base, and were used as substrates. For the PCR method, refer to Sambrook et al., "Molecular Cloning" *A Laboratory Manual,* Cold Spring Harbor Laboratory Press, 1989. After subjecting a protein and these substrates to reaction in a buffer solution containing γ-[³²P]-ATP, the reaction solution was separated by electrophoresis (SDS-PAGE), the resulting gel was dried, and the phosphorylated substrate band was detected by autoradiography.

Results showed that when GST-Cdc25C was used as a substrate, the hCdr2 wt protein phosphorylated GST-Cdc25C (C terminal peptide: 195-260aa) but failed to phosphorylate the hCdr2 K59A protein. When GST-Cdc25C S216A was used as a substrate, phosphorylation of the hCdr2 wt protein did not occur. These results indicate that hCdr2 is a protein kinase that phosphorylates S216 of Cdc25C.

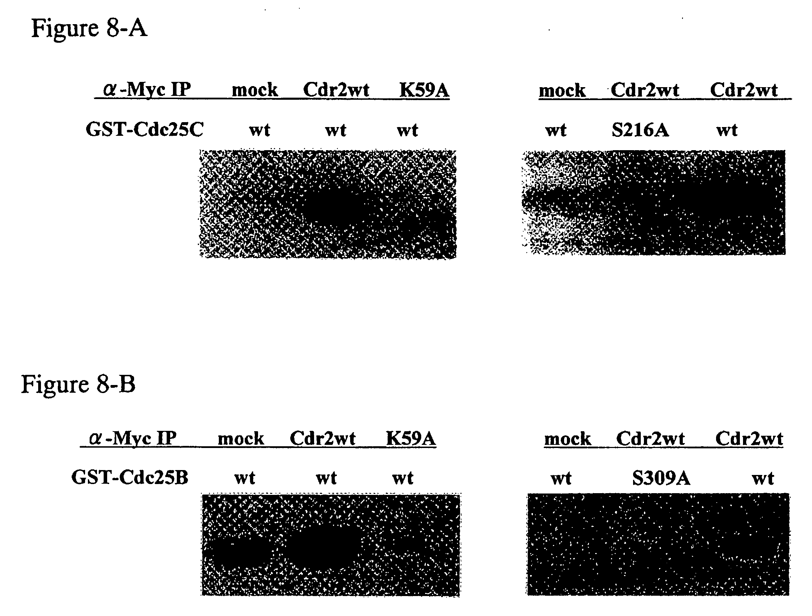

When GST-Cdc25B was used as a substrate, the hCdr2 wt protein severely phosphorylated the GST-Cdc25B (C terminal peptide: 282-381aa) as opposed to the weak phosphorylating action exhibited by the mock and hCdr2 K59A. When GST-Cdc25B S309A was used as a substrate, even the hCdr2 wt protein did not provide phosphorylation action (Figure 8-B). These results show that hCdr2 is a protein kinase that phosphorylates S309 of Cdc25B.

As described above, hCdr2 did not exhibit kinase activity with respect to these mutagenetic substrates. It is thus established that regions of Cdc25C and Cdc25B that are phosphorylated by hCdr2 *in vitro* include the 216^{th} and 309^{th} serines, respectively. Moreover, since the mutated protein hCdr2 K59A found at the ATP binding site of hCdr2 was not capable of phosphorylating Cdc25C and Cdc25B peptides, it was verified that the phosphorylation measured in the kinase assay is derived from the wt recombinant hCdr2.

### Example 6: Intracellular locality

To analyze the locality of hCdr2 in a cell, the hCdr2 gene was inserted into a pEGFP-N1 vector (Genebank Accession No. U55762), and GFP-fused hCdr2 was transient-transfected into Hela cells using TransIT-LT1 (produced by Mirus Corporation). It was confirmed by fluorescence microscopy that the GFP-hCdr2 is present in the cytoplasm and nucleus.

### INDUSTRIAL APPLICABILITY

The present invention provides a mammal-derived protein having kinase activity, and its gene. Since the protein of the invention is considered to be involved in cell cycle regulation, the present invention enables the development of novel pharmaceuticals to diagnose and treat proliferative disorders such as cancer and the like. Additionally, the gene that codes for the instant protein can be applied to the gene therapy of the aforementioned diseases. The present invention also provides a host-vector system for producing the instant protein, and thereby enabling the instant protein to be mass-produced. Furthermore, the invention provides an oligonucleotide that specifically hybridizes to the DNA encoding the instant protein, and an antibody that binds to the instant protein, thereby enabling the instant protein and its gene to be readily detected and isolated. The present invention further provides compounds that bind to the instant protein and a method of screening compounds that promote or inhibit the activity of the protein. Compounds thus isolated are of use as candidate pharmaceutical compounds to diagnose and treat the aforementioned diseases. Compounds that inhibit the activity and expression of the instant protein are expected to be used especially as anti-cancer agents.

## Claims

1. A protein according to (i) or (ii) below:
(i) a protein comprising the amino acid sequence of SEQ ID NO: 1; or
(ii) a protein comprising the amino acid sequence of SEQ ID NO: 1, in which one or more amino acids are substituted, deleted, inserted, and/or added, and which has kinase activity.

2. A protein that is encoded by a mammal-derived DNA that hybridizes to a DNA comprising the base sequence of SEQ ID NO: 2, and has a kinase activity.

3. The protein according to Claim 1 or 2 exhibiting a phosphorylating activity to Cdc25.

4. The protein according to Claim 1 or 2 exhibiting a phosphorylating activity to serine 216 of Cdc25C.

5. The protein according to Claim 1 or 2 exhibiting a phosphorylating activity to serine 309 of Cdc25B.

6. A gene encoding the protein of (i) or (ii) below:
(i) a protein comprising the amino acid sequence of SEQ ID NO: 1; or
(ii) a protein comprising the amino acid sequence of SEQ ID NO: 1, in which one or more amino acids are substituted, deleted, inserted, and/or added, and which has kinase activity.

7. A gene comprising the DNA of (i) or (ii) below:
(i) a DNA comprising the base sequence of SEQ ID NO: 2; or
(ii) a mammal-derived DNA encoding a protein that hybridizes to a DNA comprising the base sequence of SEQ ID NO: 2 and has kinase activity.

8. An expression product of the gene according to Claim 6 or 7.

9. An expression vector for a recombinant comprising the gene according to Claim 6 or 7.

10. A transformant transformed by the expression vector for the recombinant according to Claim 9.

11. A method for producing a recombinant protein, the method comprising culturing the transformant according to Claim 10 and purifying the resulting protein.

12. An oligonucleotide specifically hybridizing to the gene according to Claim 6 or 7 and comprising the chain length of at least 15 nucleotides.

13. An oligonucleotide comprising the chain length of at least 15 nucleotides that specifically hybridizes to the gene according to Claim 6 or 7 and can inhibit the expression of the gene.

14. An antibody binding to the protein according to any of Claims 1 to 5 or to the expression product according to Claim 8.

15. A method for screening a compound that has binding activity to the protein according to any of Claims 1 to 5 or to the expression product according to Claim 8, the method comprising the steps of:
(a) contacting a test sample with the protein according to any of Claims 1 to 5 or the expression product according to Claim 8; and
(b) selecting a compound that has binding activity to the protein defined in any of Claims 1 to 5 or to the expression product defined in Claim 8.

16. A method for screening a compound that can promote or inhibit kinase activity of the protein according to any of Claims 1 to 5 or the expression product according to Claim 8, the method comprising the steps of:
(a) contacting the protein according to any of Claims 1 to 5 or the expression product according to Claim 8 with a corresponding substrate in the presence of a test compound;
(b) assaying the substrate-targeted kinase activity of the protein according to any of Claims 1 to 5 or the expression product according to Claim 8; and
(c) selecting a compound that can promote or inhibit the substrate-targeted kinase activity of the protein according to any of Claims 1 to 5 or the expression product according to Claim 8, by comparing the activity with that obtained in an assay in the absence of the test compound (control).

17. A compound that can be isolated by the method according to Claim 15 or 16.

18. A compound according to Claim 17, wherein the compound is a protein.

19. A compound according to Claim 18, wherein the protein is an antibody.

20. A pharmaceutical composition comprising a compound that inhibits kinase activity of the protein according to any of Claims 1 to 5 or the expression product according to Claim 8.

21. A pharmaceutical composition comprising a compound that inhibits in a cell the expression of the gene according to Claim 6 or 7.

22. A pharmaceutical composition according to Claim 20 or 21, wherein the pharmaceutical composition is an anti-cancer agent.

23. A use of the pharmaceutical composition according to any of Claims 20 to 22 to obtain a pharmaceutical for cancer treatment.

24. A method for treating a cancer comprising administering to a cancer patient a therapeutically effective amount of the pharmaceutical composition defined in any of Claims 20 to 22.

25. A pharmaceutical composition comprising a compound that promotes kinase activity of the protein defined in any of Claims 1 to 5 or the expression product defined in Claim 8.

26. A pharmaceutical composition comprising a compound that promotes in a cell the expression of the gene defined in Claim 6 or 7.

27. A pharmaceutical composition defined in Claim 25 or 26, wherein the pharmaceutical composition is a vulnerary.

28. A use of the pharmaceutical composition defined in any of Claims 25 to 27 to obtain a pharmaceutical for healing a wound.

29. A method for healing a wound comprising administering to a patient having a wound a curatively effective amount of the pharmaceutical composition defined in any of Claims 25 to 27.


SEQUENCE LISTING

<110> TAIHO PHARMACEUTICAL CO., LTD

<120> CELL CYCLE REGULATORY FACTOR

<130> 44B00JP

<140>
<141>

<150> JP 2001-168792
<151> 2001-6-4

<160> 2
<170> PatentIn Ver. 2.1

<210> 1
<211> 754
<212> PRT
<213> Homo sapiens

<400> 1
Met Ser Ser Gly Ala Lys Glu Gly Gly Gly Gly Ser Pro Ala Tyr His
 1               5                  10                  15

Leu Pro His Pro His Pro His Pro Pro Gln His Ala Gln Tyr Val Gly
            20                  25                  30

Pro Tyr Arg Leu Glu Lys Thr Leu Gly Lys Gly Gln Thr Gly Leu Val
        35                  40                  45

Lys Leu Gly Val His Cys Ile Thr Gly Gln Lys Val Ala Ile Lys Ile
    50                  55                  60

Val Asn Arg Glu Lys Leu Ser Glu Ser Val Leu Met Lys Val Glu Arg
65                  70                  75                  80

Glu Ile Ala Ile Leu Lys Leu Ile Glu His Pro His Val Leu Lys Leu
                85                  90                  95

His Asp Val Tyr Glu Asn Lys Lys Tyr Leu Tyr Leu Val Leu Glu His
            100                 105                 110

Val Ser Gly Gly Glu Leu Phe Asp Tyr Leu Val Lys Lys Gly Arg Leu
        115                 120                 125

Thr Pro Lys Glu Ala Arg Lys Phe Phe Arg Gln Ile Val Val Cys Ala
    130                 135                 140

Gly Leu Cys His Ser Tyr Ser Ile Cys His Arg Asp Leu Lys Pro Glu
145                 150                 155                 160

Asn Leu Leu Leu Asp Glu Lys Asn Asn Ile Arg Ile Ala Asp Phe Gly
                165                 170                 175

Met Ala Ser Leu Gln Val Gly Asp Ser Leu Leu Glu Thr Ser Cys Gly
            180                 185                 190

Ser Pro His Tyr Ala Cys Pro Glu Val Ile Lys Gly Glu Lys Tyr Asp
        195                 200                 205

Gly Arg Arg Ala Asp Met Trp Ser Cys Gly Val Ile Leu Phe Ala Leu
    210                 215                 220

Leu Val Gly Ala Leu Pro Phe Asp Asp Asp Asn Leu Arg Gln Leu Leu
225                 230                 235                 240

Glu Lys Val Lys Arg Gly Val Phe His Met Pro His Phe Ile Pro Pro
                245                 250                 255

Asp Cys Gln Ser Leu Leu Arg Gly Met Ile Glu Val Glu Pro Glu Lys
            260                 265                 270

Arg Leu Ser Leu Glu Gln Ile Gln Lys His Pro Trp Tyr Leu Gly Gly
        275                 280                 285

Lys His Glu Pro Asp Pro Cys Leu Glu Pro Ala Pro Gly Arg Arg Val
    290                 295                 300

Ala Met Arg Ser Leu Pro Ser Asn Gly Glu Leu Asp Pro Asp Val Leu
305                 310                 315                 320

Glu Ser Met Ala Ser Leu Gly Cys Phe Arg Asp Arg Glu Arg Leu His
                325                 330                 335

Arg Glu Leu Arg Ser Glu Glu Glu Asn Gln Glu Lys Met Ile Tyr Tyr
            340                 345                 350

Leu Leu Leu Asp Arg Lys Glu Arg Tyr Pro Ser Cys Glu Asp Gln Asp
        355                 360                 365

Leu Pro Pro Arg Asn Asp Val Asp Pro Pro Arg Lys Arg Val Asp Ser
    370                 375                 380

Pro Met Leu Ser Arg His Gly Lys Arg Arg Pro Glu Arg Lys Ser Met
385                 390                 395                 400

Glu Val Leu Ser Ile Thr Asp Ala Gly Gly Gly Gly Ser Pro Val Pro
                405                 410                 415

Thr Arg Arg Ala Leu Glu Met Ala Gln His Ser Gln Arg Ser Arg Ser
            420                 425                 430

Val Ser Gly Ala Ser Thr Gly Leu Ser Ser Ser Pro Leu Ser Ser Pro
        435                 440                 445

Arg Ser Pro Val Phe Ser Phe Ser Pro Glu Pro Gly Ala Gly Asp Glu
    450                 455                 460

Ala Arg Gly Gly Gly Ser Pro Thr Ser Lys Thr Gln Thr Leu Pro Gly
465                 470                 475                 480

Pro Pro Gly Ser Pro Arg Ser Ser Gly Gly Thr Pro Leu His Ser Pro
                485                 490                 495

Leu His Thr Pro Arg Ala Ser Pro Thr Gly Thr Pro Gly Thr Thr Pro
            500                 505                 510

Pro Pro Ser Pro Gly Gly Gly Val Gly Gly Ala Ala Trp Arg Ser Arg
        515                 520                 525

Leu Asn Ser Ile Arg Asn Ser Phe Leu Gly Ser Pro Arg Phe His Arg
    530                 535                 540

Arg Lys Met Gln Val Pro Thr Ala Glu Glu Met Ser Ser Leu Thr Pro
545                 550                 555                 560

Glu Ser Ser Pro Glu Leu Ala Lys Arg Ser Trp Phe Gly Asn Phe Ile
                565                 570                 575

Ser Leu Asp Lys Glu Glu Gln Ile Phe Leu Val Leu Lys Asp Lys Pro
            580                 585                 590

Leu Ser Ser Ile Lys Ala Asp Ile Val His Ala Phe Leu Ser Ile Pro
        595                 600                 605

Ser Leu Ser His Ser Val Leu Ser Gln Thr Ser Phe Arg Ala Glu Tyr
    610                 615                 620

Lys Ala Ser Gly Gly Pro Ser Val Phe Gln Lys Pro Val Arg Phe Gln
625                 630                 635                 640

Val Asp Ile Ser Ser Ser Glu Gly Pro Glu Pro Ser Pro Arg Arg Asp
                645                 650                 655

Gly Ser Gly Gly Gly Gly Ile Tyr Ser Val Thr Phe Thr Leu Ile Ser
            660                 665                 670

Gly Pro Ser Arg Arg Phe Lys Arg Val Val Glu Thr Ile Gln Ala Gln
        675                 680                 685

Leu Leu Ser Thr His Asp Gln Pro Ser Val Gln Ala Leu Ala Asp Glu
    690                 695                 700

Lys Asn Gly Ala Gln Thr Arg Pro Ala Gly Ala Pro Pro Arg Ser Leu
705                 710                 715                 720

Gln Pro Pro Pro Gly Arg Pro Asp Pro Glu Leu Ser Ser Ser Pro Arg
                725                 730                 735

Arg Gly Pro Pro Lys Asp Lys Lys Leu Leu Ala Thr Asn Gly Thr Pro
            740                 745                 750

Leu Pro

<210> 2
<211> 2799
<212> DNA
<213> Homo sapiens

<220>
<221> CDS
<222> (50)..(2311)

<400> 2
gcggccgcgt cgacgccggg ggaccggtcg ggccgggacc aagggcacc atg tcg tcc 58

Met Ser Ser
1

ggg gcc aag gag gga ggt ggg ggc tct ccc gcc tac cac ctc ccc cac 106
Gly Ala Lys Glu Gly Gly Gly Gly Ser Pro Ala Tyr His Leu Pro His
    5                   10                  15

ccc cac ccc cac cca ccc cag cac gcc caa tat gtg ggc ccc tat cgg 154
Pro His Pro His Pro Pro Gln His Ala Gln Tyr Val Gly Pro Tyr Arg
20                  25                  30                  35

ctg gag aag acg ctg ggc aaa gga cag aca ggg ctg gtt aaa ctc ggg 202
Leu Glu Lys Thr Leu Gly Lys Gly Gln Thr Gly Leu Val Lys Leu Gly
                40                  45                  50

gtc cac tgc atc acg ggt cag aag gtc gcc atc aag atc gtg aac cgg 250
Val His Cys Ile Thr Gly Gln Lys Val Ala Ile Lys Ile Val Asn Arg
            55                  60                  65

gag aag ctg tcg gag tcg gtg ctg atg aag gtg gag cgg gag atc gcc 298
Glu Lys Leu Ser Glu Ser Val Leu Met Lys Val Glu Arg Glu Ile Ala
        70                  75                  80

atc ctg aag ctc atc gaa cac cca cat gtc ctc aag ctc cac gac gtc 346
Ile Leu Lys Leu Ile Glu His Pro His Val Leu Lys Leu His Asp Val
    85                  90                  95

tac gag aac aag aaa tat ttg tac ctg gtt ctg gag cac gtc tcg ggg 394
Tyr Glu Asn Lys Lys Tyr Leu Tyr Leu Val Leu Glu His Val Ser Gly
100                 105                 110                 115

ggt gag cta ttc gac tac ctg gta aag aag ggg aga ctg acg ccc aag 442
Gly Glu Leu Phe Asp Tyr Leu Val Lys Lys Gly Arg Leu Thr Pro Lys
                120                 125                 130

gag gcc cga aag ttc ttc cgc cag att gtt gtc tgc gct gga ctc tgc 490

Glu Ala Arg Lys Phe Phe Arg Gln Ile Val Val Cys Ala Gly Leu Cys
            135                 140                 145

cac agc tac tcc atc tgc cac aga gac cta aag ccc gag aac ctg ctt 538
His Ser Tyr Ser Ile Cys His Arg Asp Leu Lys Pro Glu Asn Leu Leu
        150                 155                 160

ttg gat gag aaa aac aac atc cgc att gca gac ttc ggc atg gcg tcc 586
Leu Asp Glu Lys Asn Asn Ile Arg Ile Ala Asp Phe Gly Met Ala Ser
    165                 170                 175

ctg cag gtg ggg gac agc ctc ctg gag acc agc tgc ggg tcc ccc cat 634
Leu Gln Val Gly Asp Ser Leu Leu Glu Thr Ser Cys Gly Ser Pro His
180                 185                 190                 195

tat gcg tgt cca gag gtg att aag ggg gaa aaa tat gat ggc cgc cgg 682
Tyr Ala Cys Pro Glu Val Ile Lys Gly Glu Lys Tyr Asp Gly Arg Arg
                200                 205                 210

gca gac atg tgg agc tgt gga gtc atc ctc ttc gcc ctg ctc gtg ggg 730
Ala Asp Met Trp Ser Cys Gly Val Ile Leu Phe Ala Leu Leu Val Gly
            215                 220                 225

gct ctg ccc ttt gat gac gac aac ctc cgc cag ctg ctg gag aag gtg 778
Ala Leu Pro Phe Asp Asp Asp Asn Leu Arg Gln Leu Leu Glu Lys Val
        230                 235                 240

aaa cgg ggc gtc ttc cac atg ccc cac ttc att cct cca gat tgc cag 826
Lys Arg Gly Val Phe His Met Pro His Phe Ile Pro Pro Asp Cys Gln
    245                 250                 255

agc ctc ctg agg gga atg atc gaa gtg gag ccc gaa aaa agg ctc agt 874
Ser Leu Leu Arg Gly Met Ile Glu Val Glu Pro Glu Lys Arg Leu Ser
260                 265                 270                 275

ctg gag caa att cag aaa cat cct tgg tac cta ggc ggg aaa cac gag 922

Leu Glu Gln Ile Gln Lys His Pro Trp Tyr Leu Gly Gly Lys His Glu
                280                 285                 290

cca gac ccg tgc ctg gag cca gcc cct ggc cgc cgg gta gcc atg cgg 970
Pro Asp Pro Cys Leu Glu Pro Ala Pro Gly Arg Arg Val Ala Met Arg
            295                 300                 305

agc ctg cca tcc aac gga gag ctg gac ccc gac gtc cta gag agc atg 1018
Ser Leu Pro Ser Asn Gly Glu Leu Asp Pro Asp Val Leu Glu Ser Met
        310                 315                 320

gca tca ctg ggc tgc ttc agg gac cgc gag agg ctg cat cgc gag ctg 1066
Ala Ser Leu Gly Cys Phe Arg Asp Arg Glu Arg Leu His Arg Glu Leu
    325                 330                 335

cgc agt gag gag gag aac caa gaa aag atg ata tat tat ctg ctt ttg 1114
Arg Ser Glu Glu Glu Asn Gln Glu Lys Met Ile Tyr Tyr Leu Leu Leu
340                 345                 350                 355

gat cgg aag gag cgg tat ccc agc tgt gag gac cag gac ctg cct ccc 1162
Asp Arg Lys Glu Arg Tyr Pro Ser Cys Glu Asp Gln Asp Leu Pro Pro
                360                 365                 370

cgg aat gat gtt gac ccc ccc cgg aag cgt gtg gat tct ccc atg ctg 1210
Arg Asn Asp Val Asp Pro Pro Arg Lys Arg Val Asp Ser Pro Met Leu
            375                 380                 385

agc cgt cac ggg aag cgg cga cca gag cgg aag tcc atg gaa gtc ctg 1258
Ser Arg His Gly Lys Arg Arg Pro Glu Arg Lys Ser Met Glu Val Leu
        390                 395                 400

agc atc acc gat gcc ggg ggt ggt ggc tcc cct gta ccc aca cga cgg 1306
Ser Ile Thr Asp Ala Gly Gly Gly Gly Ser Pro Val Pro Thr Arg Arg
    405                 410                 415

gcc ttg gag atg gcc cag cac agc cag aga tcc cgt agc gtc agt gga 1354

Ala Leu Glu Met Ala Gln His Ser Gln Arg Ser Arg Ser Val Ser Gly
420                 425                 430                 435

gcc tcc acg ggt ctg tcc tcc agc cct cta agc agc cca agg agt ccg    1402
Ala Ser Thr Gly Leu Ser Ser Ser Pro Leu Ser Ser Pro Arg Ser Pro
                440                 445                 450

gtc ttt tcc ttt tca ccg gag ccg ggg gct gga gat gag gct cga ggc    1450
Val Phe Ser Phe Ser Pro Glu Pro Gly Ala Gly Asp Glu Ala Arg Gly
            455                 460                 465

ggg ggc tcc ccg act tcc aaa acg cag acg ctg ccc ggc ccc cca ggc    1498
Gly Gly Ser Pro Thr Ser Lys Thr Gln Thr Leu Pro Gly Pro Pro Gly
        470                 475                 480

tcc ccg cgc tcc tct ggc ggg acc ccc ttg cac tcg cct ctg cac acg    1546
Ser Pro Arg Ser Ser Gly Gly Thr Pro Leu His Ser Pro Leu His Thr
    485                 490                 495

ccc cgg gcc agt ccc acc ggg acc ccg ggg aca aca cca ccc ccc agc    1594
Pro Arg Ala Ser Pro Thr Gly Thr Pro Gly Thr Thr Pro Pro Pro Ser
500                 505                 510                 515

ccc ggc ggt ggc gtc ggg ggg gcc gcc tgg aga agt cgt ctc aac tcc    1642
Pro Gly Gly Gly Val Gly Gly Ala Ala Trp Arg Ser Arg Leu Asn Ser
                520                 525                 530

atc cgc aac agc ttc ctg ggc tcc cct cgc ttt cac cgg cgc aag atg    1690
Ile Arg Asn Ser Phe Leu Gly Ser Pro Arg Phe His Arg Arg Lys Met
            535                 540                 545

cag gtc cct acc gct gag gag atg tcc agc ttg acg cca gag tcc tcc    1738
Gln Val Pro Thr Ala Glu Glu Met Ser Ser Leu Thr Pro Glu Ser Ser
        550                 555                 560

ccg gag ctg gca aaa cgc tcc tgg ttc ggg aac ttc atc tcc ttg gac    1786

Pro Glu Leu Ala Lys Arg Ser Trp Phe Gly Asn Phe Ile Ser Leu Asp
    565                 570                 575

aaa gaa gag caa ata ttc ctc gtg cta aag gac aaa cct ctc agc agc 1834
Lys Glu Glu Gln Ile Phe Leu Val Leu Lys Asp Lys Pro Leu Ser Ser
580                 585                 590                 595

atc aaa gca gac atc gtc cat gcc ttt ctg tcg atc ccc agc ctg agt 1882
Ile Lys Ala Asp Ile Val His Ala Phe Leu Ser Ile Pro Ser Leu Ser
                600                 605                 610

cac agt gtg ctg tca cag acc agc ttc agg gcc gag tac aag gcc agt 1930
His Ser Val Leu Ser Gln Thr Ser Phe Arg Ala Glu Tyr Lys Ala Ser
            615                 620                 625

ggc ggc ccc tcc gtc ttc caa aag ccc gtc cgc ttc cag gtg gac atc 1978
Gly Gly Pro Ser Val Phe Gln Lys Pro Val Arg Phe Gln Val Asp Ile
        630                 635                 640

agc tcc tct gag ggt cca gag ccc tcc ccg cga cgg gac ggc agc gga 2026
Ser Ser Ser Glu Gly Pro Glu Pro Ser Pro Arg Arg Asp Gly Ser Gly
    645                 650                 655

ggt ggt ggc atc tac tcc gtc acc ttc act ctc atc tcg ggt ccc agc 2074
Gly Gly Gly Ile Tyr Ser Val Thr Phe Thr Leu Ile Ser Gly Pro Ser
660                 665                 670                 675

cgt cgg ttc aag cga gtg gtg gag acc atc cag gca cag ctc ctg agc 2122
Arg Arg Phe Lys Arg Val Val Glu Thr Ile Gln Ala Gln Leu Leu Ser
                680                 685                 690

act cat gac cag ccc tcc gtg cag gcc ctg gca gac gag aag aac ggg 2170
Thr His Asp Gln Pro Ser Val Gln Ala Leu Ala Asp Glu Lys Asn Gly
            695                 700                 705

gcc cag acc cgg cct gct ggt gcc cca ccc cga agc ctg cag ccc cca 2218

Ala Gln Thr Arg Pro Ala Gly Ala Pro Pro Arg Ser Leu Gln Pro Pro
        710                 715                 720

ccc ggc cgc cca gac cca gag ctg agc agc tct ccc cgc cga ggc ccc    2266
Pro Gly Arg Pro Asp Pro Glu Leu Ser Ser Ser Pro Arg Arg Gly Pro
    725                 730                 735

ccc aag gac aag aag ctc ctg gcc acc aac ggg acc cct ctg ccc        2311
Pro Lys Asp Lys Lys Leu Leu Ala Thr Asn Gly Thr Pro Leu Pro
740                 745                 750

tgaccccacg gggccgggga gggaggggac ccccctccac cccccttccg tgccccccaa 2371

ctgtgaatct gtaaataagg cccaaggaac atgtcgggag gggggtggac acaaaaaccg 2431

gccttgccct gcagggatgg ggctccacag gccgtgccca actgggggtg gttctagggg 2491

aacagggggc gggggagctg tttctatttt atttattgat taatttatta ttttatttat 2551

tgatcaatct ctctgcgggg tggggtgggg gagggacggg agctggttgg ggtggcttag 2611

cagatccgga cagggccctc tgtccctgtg tcgtccccaa ccccctcttc ccgggcccct 2671

cctcccctgg tcctcccccc acgaccttct gtacggattt gctctccgga aggaattctg 2731

gtttcgcgtg atcctgcctg cgtccgtgtc tctgattccg ccggcggcaa aaaaaaaaaa 2791

aaaaaaaa                                                          2799